(19) Europäisches Patentamt
European Patent Office
Office européen des brevets

(11) Publication number: 0 355 710 A2

# (12) EUROPEAN PATENT APPLICATION

(21) Application number: 89115178.9

(22) Date of filing: 17.08.89

(51) Int. Cl.[4]: C07D 333/24 , C07D 307/54 , A61K 31/38 , A61K 31/34

Claims for the following Contracting States: ES + GR.

(30) Priority: 22.08.88 US 234991

(43) Date of publication of application: 28.02.90 Bulletin 90/09

(84) Designated Contracting States: AT BE CH DE ES FR GB GR IT LI LU NL SE

(71) Applicant: MERRELL DOW PHARMACEUTICALS INC.
P.O. Box 156300 2110 East Galbraith Road
Cincinnati Ohio 45215-6300(US)

(72) Inventor: Sabol, Jeffrey S.
1843 Poplar Drive
Loveland Ohio 45140(US)

(74) Representative: Vossius & Partner
Siebertstrasse 4 P.O. Box 86 07 67
D-8000 München 86(DE)

## (54) Phenylalkylthiophenes and related compounds.

(57) The present invention is directed to phenylalkylthienylalkanoic acids and related compounds having a mercaptoalkanoic acid substituent. These compounds are useful in the treatment of bronchial asthma and they are obtained by the reaction of an appropriate epoxy compound with a mercapto alkanoic acid. Both reactants in this process are used as the ester so that the reaction gives a diester which is then hydrolyzed to the corresponding free acids by standard procedures.

EP 0 355 710 A2

## PHENYLALKYLTHIOPHENES AND RELATED COMPOUNDS

The present invention relates to a group of phenylalkyl thiophenes which are further substituted by a thiadicarboxylic acid group. More particularly, the present invention relates to compounds having the following general formula:

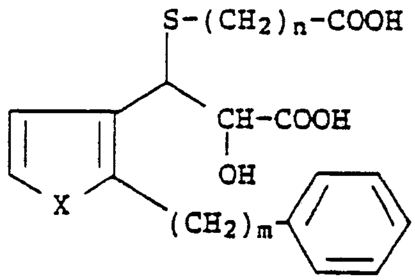

or

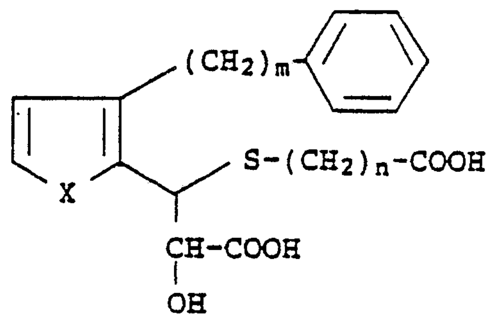

wherein X is S or O, m is an integer from 6 to 10 and n is an integer from 1 to 4.

Where stereoisomerism is possible with regard to the present compounds, the chemical structures as presented above are considered as encompassing all of the possible stereoisomers and also racemic mixtures of such stereoisomers.

The therapeutically acceptable salts of the present acids are also included within the scope of the present invention. These basic salts would include, but would not be limited to, sodium, potassium, calcium, magnesium, triethylamine, tromethamine, dicyclohexylamine and the like as is well-known in the art. Such base salts can be obtained be standard procedures using the free acids of the present invention and the appropriate base. The preferred compounds of the present invention, however, are the free acids.

As examples of the compounds of the present invention are the following:

3-[(2-Carboxyethyl)thio]-3-[[2-(8-phenyloctyl]-3-thienyl]-2-hydroxypropanoic acid.
3-[(2-Carboxyethyl)thio]-3-[[2-(6-phenylhexyl]-3-thienyl]-2-hydroxypropanoic acid.
3-[(2-Carboxyethyl)thio]-3-[[2-(10-phenyldecyl]-3-thienyl]-2-hydroxypropanoic acid.
3-[(Carboxymethyl)thio]-3-[[2-(8-phenyloctyl]-3-thienyl]-2-hydroxypropanoic acid.
3-[(3-Carboxypropyl)thio]-3-[[2-(8-phenyloctyl]-3-thienyl]-2-hydroxypropanoic acid.
3-[(4-Carboxybutyl)thio]-3-[[2-(8-phenyloctyl]-3-thienyl]-2-hydroxypropanoic acid.
3-[(2-Carboxyethyl)thio]-3-[[3-(8-phenyloctyl]-2-thienyl]-2-hydroxypropanoic acid.
3-[(2-Carboxyethyl)thio]-3-[[3-(6-phenylhexyl]-2-thienyl]-2-hydroxypropanoic acid.
3-[(2-Carboxyethyl)thio]-3-[[3-(10-phenyldecyl]-2-thienyl]-2-hydroxypropanoic acid.
3-[(Carboxymethyl)thio]-3-[[3-(8-phenyloctyl]-2-thienyl]-2-hydroxypropanoic acid.
3-[(3-Carboxypropyl)thio]-3-[[3-(8-phenyloctyl]-2-thienyl]-2-hydroxypropanoic acid.
3-[(4-Carboxybutyl)thio]-3-[[3-(8-phenyloctyl]-2-thienyl]-2-hydroxypropanoic acid.
3-[(2-Carboxyethyl)thio]-3-[[2-(8-phenyloctyl]-3-furanyl]-2-hydroxypropanoic acid.
3-[(2-Carboxyethyl)thio]-3-[[3-(8-phenyloctyl]-2-furanyl]-2-hydroxypropanoic acid.

The compounds of the present invention are prepared by reacting epoxide esters of the formula:

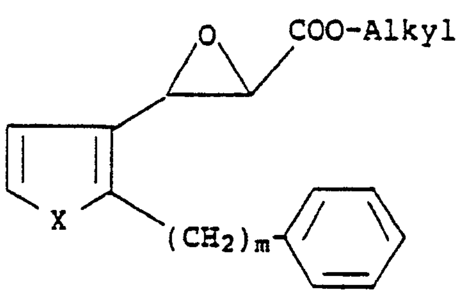

or

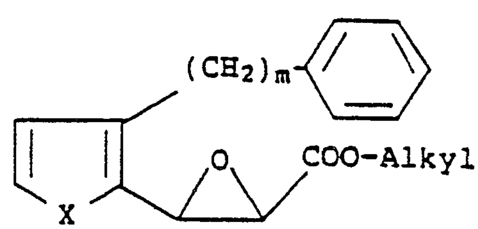

wherein X is S or O and m is an integer from 6 to 10, with a mercaptoalkanoic acid ester of the formula:

HS-$(CH_2)_n$COO-Alkyl

wherein n is an integer from 1 to 4 and the Alkyl groups contain 1 to 4 carbon atoms. This procedure gives the diesters corresponding to the compounds of the present invention and such esters are then hydrolyzed to the desired free carboxylic acids by treatment with a strong inorganic base followed by acidification with a strong acid.

The epoxide esters used in the above process are themselves prepared by reacting the corresponding thiophenecarboxaldehyde or furancarboxaldehyde with lithium bis(trimethylsilyl)amide and an alkyl bromoacetate at -70 °C. Methyl bromoacetate is preferred for this reaction.

Preparation of the starting materials used above can be illustrated by the following procedures. In the case of the 2-phenylalkyl-3-thiophenecarboxaldehydes, this starting material is obtained from the corresponding ethylene ketal by treatment with acid by standard procedures. The 2-substituted aldehyde is itself obtained from 3-thiophenecarboxaldehyde by first converting that compound to the corresponding ethylene ketal by standard procedures and then treating that ketal with butyl lithium, hexamethylphosphoramide and an appropriate phenylalkyl iodide.

For the 3-phenylalkyl-2-thiophenecarboxaldehydes, it is preferred to start with the known 4,4-dimethyl-2-(2-thienyl)-2-oxazoline and introduce the phenylalkyl group at the 3-position by procedures similar to that described above for the 2-phenylalkyl compound. The resulting oxazoline is then methylated with methyl iodide, reduced to the corresponding oxazolidine by means of a hydride reducing agent, and then finally acidified to give the desired aldehyde.

The compounds of the present invention are useful in the treatment of allergic diseases and, particularly, in the treatment of bronchial asthma. Thus, SRS-A (slow-reacting substance of anaphylaxis) is known as a substance which is a very important mediator in allergic bronchial asthma. Specifically, SRS-A is a substance which is synthesized and released in or near target tissues, in a sensitive allergic subject, shortly after challenge by the appropriate antigen with the human bronchus being particularly sensitive to SRS-A. Thus, a substance which would counteract the effects of SRS-A would be useful in the treatment of bronchial asthma.

More recent studies have established that SRS-A is actually a mixture of substances which can be described as peptido-leukotrienes. $LTD_4$ is one of these leukotrienes and can be considered as representative of them so that antagonism of this specific substance would provide effects similar to the antagonism of SRS-A generally. Specifically, the compounds of the present invention are useful as antagonists of $LTD_4$ so that they are useful in the treatment of allergic diseases and, particularly in the treatment of bronchial asthma. The present compounds are selective in this antagonist activity in that they are not considered as competitive against histamine or carbachol. The present compounds are further particularly useful in that the antagonist activity is not accompanied by simultaneous agonist activity. That is, the present compounds show a good separation of these two effects.

The activity of the compounds of the present invention can be demonstrated by the following test procedures.

## Longitudinal Muscle of Guinea Pig Ileum

Male, Hartley-Duncan guinea pigs were sacrificed by cervical dislocation. The terminal portion of the ileum was removed, rinsed, and placed in Burn's modified Tyrode's solution. The longitudinal muscle was then carefully dissected from the circular muscle of the ileum. The longitudinal muscle was cut into 1-2 cm. segments which were placed in a tissue bath containing oxygenated Burn's modified Tyrode's solution warmed to 37 °C. A tension of 1.0 gram was then placed on each segment of muscle. After equilibration for 1 hour, 1 µM Indomethacin was added to each bath. After 5 minutes, each tissue segment was then exposed to a concentration of 60nM leukotriene $D_4$. This response was then considered to be the initial maximal contraction that each segment will produce. After washing the tissue several times, over a 1 hour period, 1 µM Indomethacin was again added to each bath. After a 5 minute period, the test agent or vehicle was added to the bath. After 15 minutes, a concentration-response curve was generated using cumulatively increasing concentrations of leukotriene $D_4$. The concentration-response was then compared to the initial maximum contraction. A test compound was considered active, if at concentrations up to 100 µM, it produces a significant shift to the right of the concentration-response relationship to leukotriene $D_4$. The antagonist activity was quantitated in terms of a $pA_2$ value calculated according to the method described by Arunlakshana and Schild [Brit. J. Pharmac. Chemotherap. 14, 48 (1959)]. When 3-[(2-carboxyethyl)thio]-3-[-[2-(8-phenyloctyl]-3-thienyl]-2-hydroxypropanoic acid was tested by the above procedure, it showed a pA of

8.4.

## $^3$H-LTD$_4$-Specific Receptor Binding In Guinea Pig Lung Membranes

Male guinea pigs were sacrificed and the lungs were removed and placed in cold 50mM Tris-HCl buffer, pH 7.4. The lungs were then homogenized with a Polytron homogenizer and the homogenate was centrifuged at 1000 g for 10 minutes at 4° C. The supernatant was then centrifuged at 30,000 g for 15 minutes at 4° C. to obtain the membrane pellet. This pellet was resuspended in 50mM Tris-HCl to provide the working suspension of lung membranes. Binding assays were then carried out in 50mM Tris-HCl at pH 7.6 and 37° C. using incubation periods of 20-40 min. Separation of bound $^3$H-LTD$_4$ from free $^3$H-LTD$_4$ were performed by rapid vacuum filtration through Whatman GF/B glass fiber filters using ice cold Tris-HCl buffer and three 4 ml washes. Filtration and washing were completed in less than 8 seconds. The radioactivity on the filters was then measured. Specific binding of $^3$H-LTD$_4$ was defined as the binding of $^3$H-LTD$_4$ in the absence of unlabeled LTD$_4$ minus the binding of $^3$H-LTD$_4$ in the presence of 2 x $10^{-7}$ M unlabeled LTD$_4$. Specific binding of $^3$H-LTD$_4$ was 60-80% of total binding. Studies with test agents demonstrate the ability of the test compound to inhibit the specific binding of $^3$H-LTD$_4$. In these tests, increasing concentrations of the agent are used to block the $^3$H-LTD$_4$ specific binding. The concentration that reduces the specific binding of $^3$H-LTD$_4$ by 50% is termed the $IC_{50}$.

## In Vivo Biological Activity

In vivo leukotriene D$_4$ antagonist activity was determined in anesthetized guinea pigs using a modified Konzett-Rossler preparation. Specifically, the guinea pigs were anesthetized with sodium pentobarbital and surgically prepared for artificial ventilation by a constant volume respirator. The inflation pressure produced by the respirator was measured for each inflation of the guinea pigs' lungs. Increases in inflation pressure above baseline were indicative of bronchoconstriction. After establishing a baseline inflation pressure, bronchoconstriction was induced by an intravenous challenge with 100 mg/kg leukotriene D$_4$. This response was considered the initial response. After the inflation pressure returned to baseline, the guinea pig was treated with 0.5 mg/kg of test compound or vehicle, given intravenously (iv), and rechallenged with leukotriene D$_4$ at 1, 10 and 20 minutes following administration of the test compound. This response was then compared to the initial response and the % inhibition of the response was determined. When 3-[(2-carboxyethyl)thio]-3-[[2-(8-phenyloctyl)]-3-thienyl]-2-hydroxypropanoic acid was tested in this way, the following results were observed.

| TIME | PERCENT INHIBITION |
|---|---|
| 1 minute | 88 |
| 10 minutes | 48 |
| 20 minutes | 44 |

The compounds of the present invention may be administered either as individual therapeutic agents or as mixtures with other therapeutic agents. They may be administered alone but are generally administered in the form of pharmaceutical compositions, i.e., mixtures of the active agents with suitable pharmaceutical carriers or diluents. Examples of such compositions include tablets, lozenges, capsules, powders, aerosol sprays, aqueous or oily suspensions, syrups, elixirs and aqueous solutions for injection. The compounds are most preferably administered in oral dosage forms.

The nature of the pharmaceutical composition and the pharmaceutical carrier or diluent will, of course, depend on the desired route of administration, i.e., orally, parenterally or by inhalation. Oral compositions may be in the form of tablets or capsules and may contain conventional excipients such as binding agents (e.g., syrup, acacia, gelatin, sorbitol, tragacanth or polyvinylpyrrolidone), fillers (e.g., lactose, sugar, maize-starch, calcium phosphate, sorbitol or glycine), lubricants (e.g., magnesium stearate, talc, polyethylene glycol or silica), disintegrants (e.g., starch) or wetting agents (e.g., sodium lauryl sulfate). Oral liquid

preparations may be in the form of aqueous or oily suspension, solutions, emulsions, syrups, elixirs, etc., or may be presented as a dry product for reconstitution with water or other suitable vehicle before use. Such liquid preparations may contain conventional additives such as suspending agents, flavoring agents, diluents, or emulsifying agents. For parenteral administration or inhalation, solutions or suspensions of a compound of the present invention with conventional pharmaceutical vehicles may be employed, e.g., as an aerosol spray for inhalation, as an aqueous solution for intravenous injection or as an oily suspension for intramuscular injection. The compounds may also be administered by means of inhalers or other devices which permit the active compounds in the form of dry powders to come into direct contact with the lungs. Procedures for the preparation of compositions as discussed above are described in standard texts, such as Remington's Pharmaceutical Sciences, Mack Publishing Company, Easton, Pennsylvania.

The compounds of the present invention or pharmaceutical compositions thereof may be administered to human asthmatic patients at dosages in the range from about 0.1 to about 40 mg/kg. Single oral doses of approximately 1-1000 mg of active ingredient and multiple oral doses totaling up to about 4000 mg/day of active ingredient can be used. When administered by inhalation, lower doses are generally given, i.e., on the order of about 0.1 of the normal dosage for the particular compound in question. These values are illustrative only, however, and the physician of course will ultimately determine the dosage most suitable for a particular patient on the basis of factors such as age, weight, diagnosis, severity of the symptoms and the particular agent to be administered.

The following examples are presented to illustrate the present invention but they should not be construed as limiting it in any way.

## EXAMPLE 1

A solution of 8.91 g of 3-thiophenecarboxaldehyde, 0.5 g of p-toluenesulfonic acid and 25 ml of ethylene glycol in 125 ml of benzene was refluxed for 18 hours under argon and any water formed was removed using a Dean-Stark trap. The mixture was then cooled, the solvents removed on a rotary evaporator and the residue was partitioned between ether and 0.1 N sodium hydroxide. The organic layer was then separated, dried over magnesium sulfate and filtered and the solvent was evaporated to give an oil. Bulb-to-bulb distillation at 80° C.(0.1 mm) gave the ethylene acetal of 3-thiophenecarboxaldehyde (81% yield). NMR ($CDCl_3$) $\delta$ 4.00 (br s, 4, $-OCH_2CH_2O-$), 5.86 (s, 1, H at 2-position of dioxolane), 7.11-7.33 (mult, 3, thiophene -H's).

## EXAMPLE 2

To a stirred solution of 1.56 g of the ethylene acetal of 3-thiophenecarboxaldehyde in 40 ml of ether under argon was added dropwise 4 ml of 2.5 M n-butyl lithium in hexane. The mixture was then refluxed for 30 minutes during which time it changed to a yellow-orange suspension. To this mixture was then added 5 ml of hexamethylphosphoramide followed by a solution of 3.16 g of 1-iodo-8-phenyloctane in 5 ml of ether, and the mixture was then stirred at room temperature for one hour. The resulting mixture was partitioned between ice/water and ether and the organic layer was separated and washed with saturated aqueous brine. The organic layer was dried over magnesium sulfate and filtered and the solvent evaporated under reduced pressure to leave a residual oil. Flash chromatography of this oil on silica gel (hexane, then 2.5% ethyl acetate/methylene chloride) afforded the ethylene acetal of 2-(8-phenyloctyl)-3-thiophenecarboxaldehyde as an oil (53.8% yield).

## EXAMPLE 3

To a solution of 1.85 g of the ethylene acetal of 2-(8-phenyloctyl)-3-thiophenecarboxaldehyde in 10 ml of tetrahydrofuran was added 10 ml of 1 N hydrochloric acid and the mixture was stirred at room temperature for one hour. The mixture was then poured into ether, the layers were separated, and the organic layer was washed with 0.2 N aqueous sodium hydroxide and water. The organic layer was separated, dried over magnesium sulfate and filtered and the solvent was evaporated under reduced

pressure to leave a residual oil. Purification of the oil by Waters Prep 500 LC (silica gel, 5% ethyl acetate/hexane) afforded the purified 2-(8-phenyloctyl)-3-thiophenecarboxaldehyde as an oil (68% yield). ms ($CI-CH_4$) 301 (M + H)

## EXAMPLE 4

To 3.3 ml of 1 M lithium bis(trimethylsilyl)amide in tetrahydrofuran was added 10 ml of tetrahydrofuran and the mixture was cooled to -70° C in a dry ice-acetone bath. To this solution was added 0.31 ml of methyl bromoacetate in 10 ml of tetrahydrofuran and stirring was continued for 30 minutes at -70° C. A solution of 1.0 g of 2-(8-phenyloctyl)-3-thiophenecarboxaldehyde in 3 ml of tetrahydrofuran was added dropwise and the mixture was warmed to 0° C and stirred at that temperature for one hour. The mixture was then poured into ice-water and thoroughly extracted with ether. The combined ether extracts were dried over magnesium sulfate and the solvent was evaporated under reduced pressure to leave an oil. Flash chromatography of the oil on silica gel (7% ethyl acetate/hexane) afforded methyl 3-[[2-(8-phenyloctyl)]-3-thienyl]-2,3-epoxypropionate as an oil (47% yield). ms ($CI-CH_4$) 373 (M + H), 313 ($M + H-HCO_2CH_3$)

## EXAMPLE 5

To a mixture of 0.41 g of 3-[[2-(8-phenyloctyl)]-3-thienyl]-2,3-epoxypropionate in 10 ml of methanol was added 0.42 ml of triethylamine followed by 0.33 ml of methyl 3-mercaptopropionate and the mixture was stirred for 24 hours at room temperature. The solvent was then removed and the residue was chromatographed on silica gel (25% ethyl acetatehexane) to give a mixture of methyl 3-[(2-carbomethoxyethyl)thio]-3-[[2-(8-phenyloctyl]-3-thienyl]-2-hydroxypropionate (34% yield) along with a quantity of its regioisomer. ms ($ClCH_4$) 493 (M + H), 373 ($M + H-HSCH_2CH_2CO_2CH_3$)

## EXAMPLE 6

To a solution of 0.18 g of methyl 3-[(2-carbomethoxyethyl)thio]-3-[[2-(8-phenylooctyl)]-3-thienyl]-2-hydroxypropionate in 2 ml of ethanol was added 0.16 g of potassium hydroxide in 1.2 ml of water and the mixture was stirred at room temperature for 5.5 hours. The mixture was then partitioned between 10 ml of ether and 10 ml of water. The aqueous layer was separated, cooled in an ice-water bath, and then acidified with 1.5 ml of 3 N hydrochloric acid. Extraction of the acidified mixture with ethyl acetate followed by removal of the solvent under reduced pressure gave an oil. Radial chromatography with 93:7:0.7 chloroform:methanol:acetic acid gave 3-[(2-carboxyethyl)thio]-3-[[2-(8-phenyloctyl)]-3-thienyl]-2-hydroxypropanoic acid as an oil (49% yield). ms ($CI-NH_3$) 482 ($M + NH_4$ +). This compound has the following structural formula:

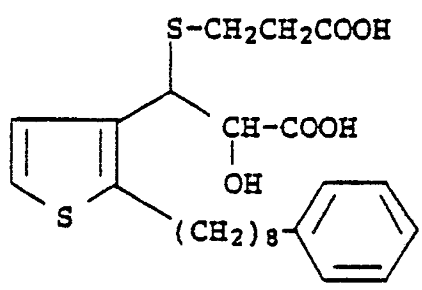

**Claims**

1. A compound of the formula

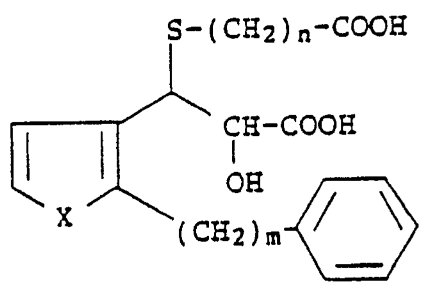

or

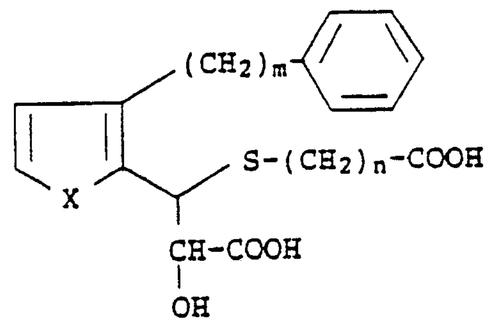

wherein X is S or O, m is an integer from 6 to 10 and n is an integer from 1 to 4.

2. A compound according to Claim 1 which has the formula

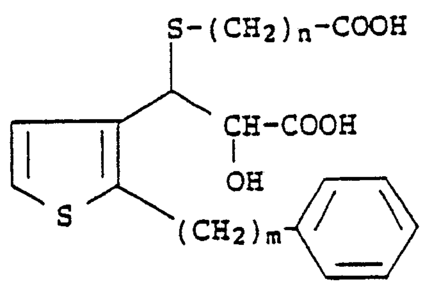

or

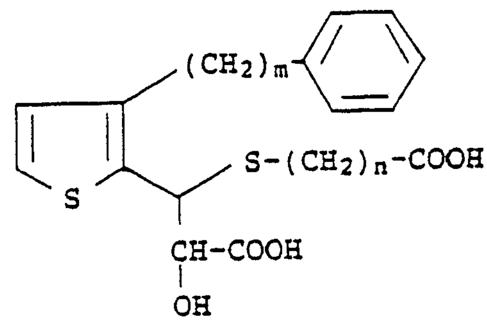

wherein m is an integer from 6 to 10 and n is an integer from 1 to 4

3. A compound according to Claim 1 which has the formula

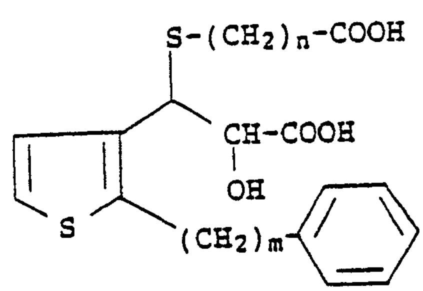

wherein m is an integer from 6 to 10 and n is an integer from 1 to 4.

4. A compound according to Claim 1 which is 3-[(2-carboxyethyl)thio-3-[[2-(8-phenyloctyl]-3-thienyl]-2-hydroxypropanoic acid.

5. A process for preparing compounds of the formula

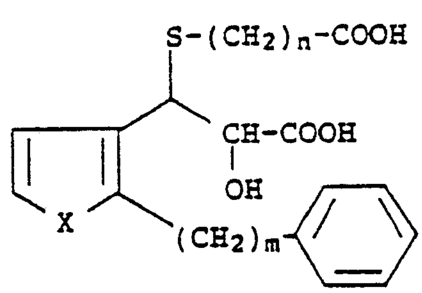

or

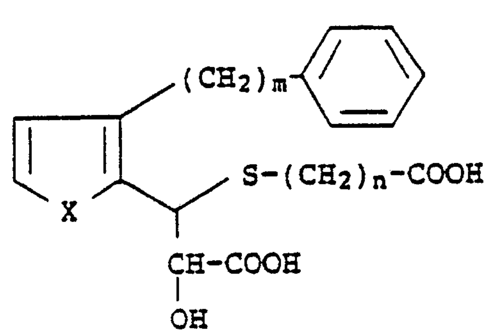

wherein X is S or O; m is an integer from 6 to 10 and n is an integer from 1 to 4, which comprises reacting compounds of the formula

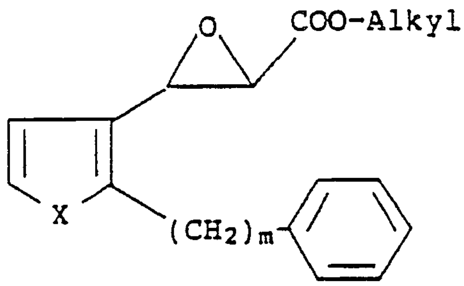

or

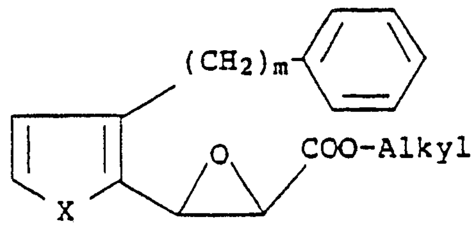

with a mercaptoalkanic acid ester of the formula
$HS-(CH_2)_nCOO-Alkyl$
followed by treatment with a strong base and then acidification with a strong acid.

6. Use of the compounds of any of claims 1 to 4 for the preparation of drugs against allergic diseases.

7. Pharmaceutical compositions comprising a pharmaceutically effective amount of a compound according to any of claims 1 to 4.

8. Pharmaceutical compositions for the treatment of allergic diseases comprising a pharmaceutically effective amount of a compound according to any of claims 1 to 4.

Claims for the following Contracting State: ES:

1. A process for preparing compounds of the formula

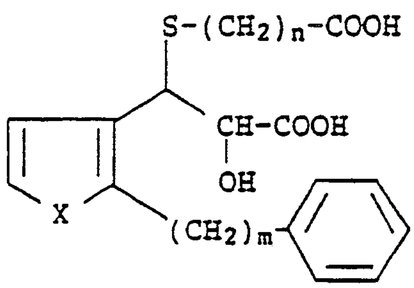

or

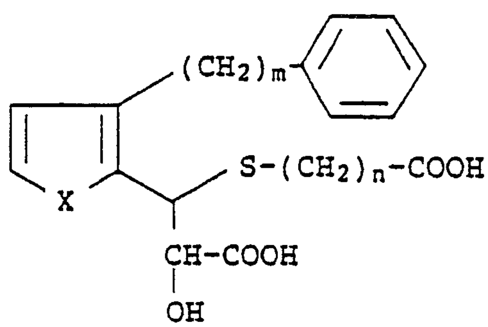

wherein X is S or O; m is an integer from 6 to 10 and n is an integer from 1 to 4, which comprises reacting compounds of the formula

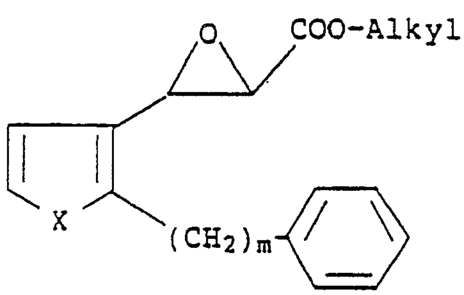

or

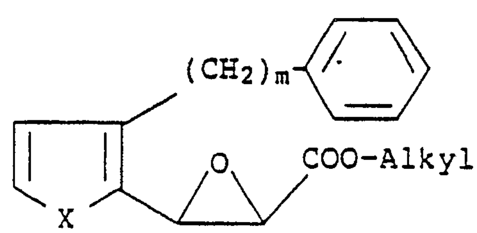

with a mercaptoalkanic acid ester of the formula
$HS-(CH_2)_nCOO-Alkyl$
followed by treatment with a strong base and then acidification with a strong acid.

2. A process according to Claim 1 for preparing compounds of the formula

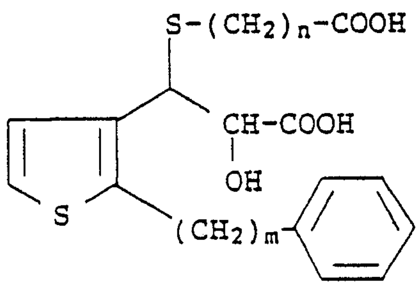

or

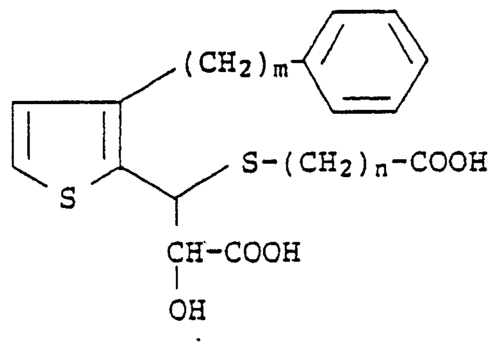

wherein m is an integer from 6 to 10 and n is an integer from 1 to 4, which comprises reacting compounds of the formula

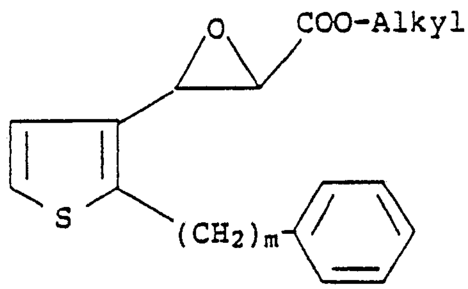

or

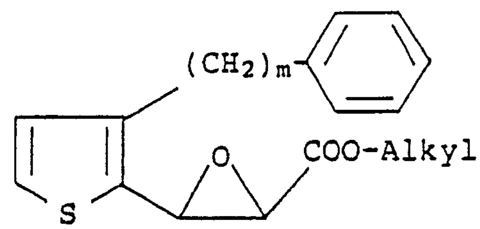

with a mercaptoalkanic acid ester of the formula

$HS-(CH_2)_nCOO-Alkyl$

followed by treatment with a strong base and then acidification with a strong acid.

3. A process according to Claim 1 for preparing a compound of the formula

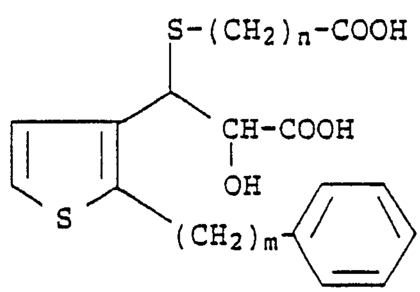

wherein m is an integer from 6 to 10 and n is an integer from 1 to 4, which comprises reacting a compound of the formula

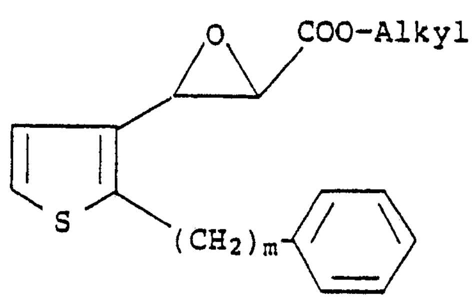

with a mercaptoalkanic acid ester of the formula

$HS-(CH_2)_nCOO-Alkyl$

followed by treatment with a strong base and then acidification with a strong acid.

4. A process according to Claim 1 for preparing 3-[(2-carboxyethyl)thio]-3-[[2-(8-phenyloctyl)]-3-thienyl]-2-hydroxypropanoic acid which comprises reacting methyl 3-[[2-(8-phenyloctyl)]-3-thienyl]-2,3-epoxypropionate with methyl 3-mercaptoproprionate followed by treatment with potassium hydroxide and then acidification with hydrochloric acid.

Claims for the following Contracting State: GR

1. A compound of the formula

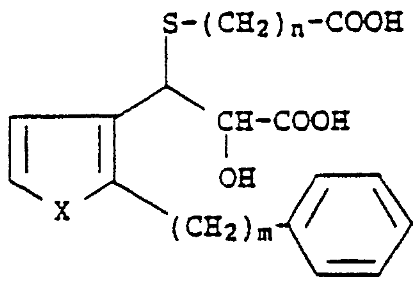

or

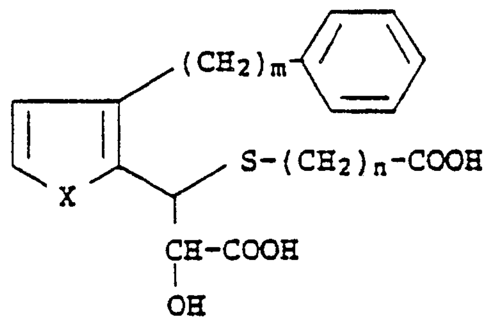

wherein X is S or O, m is an integer from 6 to 10 and n is an integer from 1 to 4.

2. A compound according to Claim 1 which has the formula

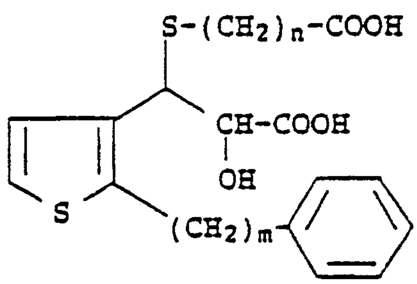

or

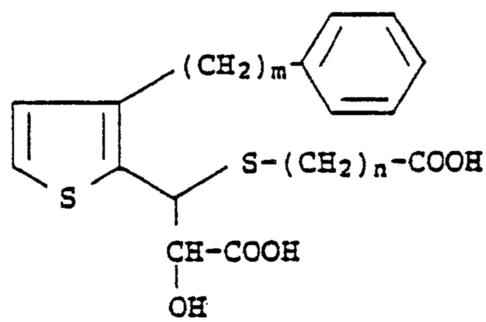

wherein m is an integer from 6 to 10 and n is an integer from 1 to 4.

3. A compound according to Claim 1 which has the formula

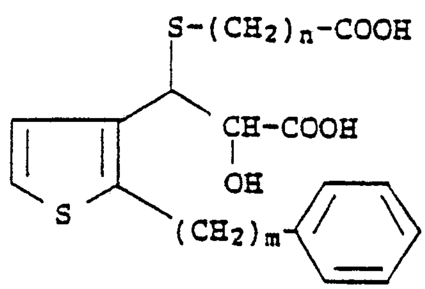

wherein m is an integer from 6 to 10 and n is an integer from 1 to 4.

4. A compound according to Claim 1 which is 3-[(2-carboxyethyl)thio]-3-[[2-(8-phenyloctyl]-3-thienyl]-2-hydroxypropanoic acid.

5. A process for preparing compounds of the formula

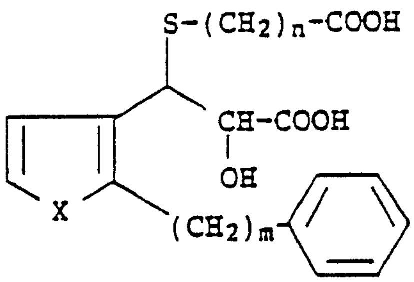

or

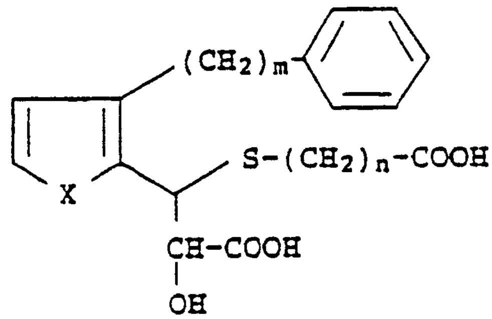

wherein X is S or O; m is an integer from 6 to 10 and n is an integer from 1 to 4, which comprises reacting compounds of the formula

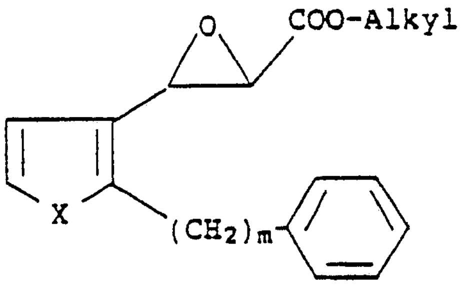

or

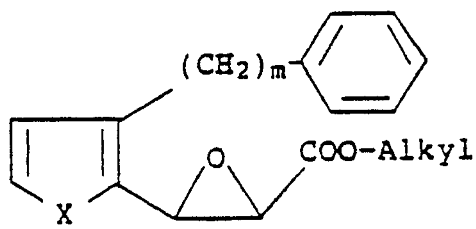

with a mercaptoalkanic acid ester of the formula
$HS-(CH_2)_nCOO-Alkyl$
followed by treatment with a strong base and then acidification with a strong acid.

6. Use of the compounds of any of claims 1 to 4 for the preparation of drugs against allergic diseases.